## Europäisches Patentamt

## European Patent Office

## Office européen des brevets

(19)

(11) Veröffentlichungsnummer: **0 034 307**

**B1**

(12) **EUROPÄISCHE PATENTSCHRIFT**

(45) Veröffentlichungstag der Patentschrift:
08.04.87

(21) Anmeldenummer: **81100803.6**

(22) Anmeldetag: **05.02.81**

(51) Int. Cl.⁴: **C 12 N 15/00,** C 12 P 21/02, C 07 H 21/04, C 12 N 1/20, C 12 P 19/34 // C12R1/19

(54) Verfahren zur Herstellung von menschlichem Leukozyten-Interferon.

(30) Priorität: 16.02.80 DE 3005897

(43) Veröffentlichungstag der Anmeldung:
26.08.81 Patentblatt 81/34

(45) Bekanntmachung des Hinweises auf die Patenterteilung:
08.04.87 Patentblatt 87/15

(84) Benannte Vertragsstaaten:
AT BE CH DE FR GB IT LI NL SE

(56) Entgegenhaltungen:
EP-A-0 018 218
EP-A-0 028 033
EP-A-0 032 134
DE-A-3 043 981
DE-A-3 103 714

Dictionary of Microbiology, John Wiley and Sons, 1978, p. 244.
RESEARCH DISCLOSURE, Nr. 183, Juli 1979, Seiten 361-362, Nr. 18309 "The production of interferon by "genetic engineering""
PROC. JAPAN. ACAD., Band 55, (9), Serie B. 12. November 1979, Seiten 464-469 T. TANIGUCHI et al.: "Construction and identification of a bacterial plasmid containing the human fibroblast interferon gene sequence"
NATURE, Band 283, 24. Januar 1980, Seite 323 P. NEWMARK: "Engineered E. coli produce

(73) Patentinhaber: **HOECHST AKTIENGESELLSCHAFT,**
**Postfach 80 03 20, D-6230 Frankfurt am Main 80**
**(DE)**

(72) Erfinder: **Groneberg, Jürgen, Dr., Loreleistrasse 71,**
**D-6230 Frankfurt am Main 80 (DE)**
Erfinder: **Wengenmayer, Friedrich, Dr., Theodor-Körner- Strasse 12, D-6238 Hofheim am Taunus (DE)**
Erfinder: **Hilfenhaus, Joachim, Dr., Auf'm Gebrande 24, D-3550 Marburg 1 (DE)**
Erfinder: **Winnacker, Ernst- Ludwig, Prof. Dr., Elvirastrasse 4, D-8000 München 19 (DE)**

(56) Entgegenhaltungen: (Fortsetzung)
interferon"
NATURE, Band 284, 27. März 1980, Seiten 316-320 S. NAGATA et al.: "Synthesis in E. coli of a polypeptide with human leukocyte interferon activity"

## Beschreibung

Seit mehr als 20 Jahren ist bekannt, daß Interferon die Virusproduktion verhindert. Untersuchungen der letzten Jahre haben ferner ergeben, daß Interferon auch die Zellteilung inhibiert und viele Reaktionen des Immunsystems reguliert. Alle diese Ergebnisse unterstreichen die große medizinische Bedeutung des Interferons.

Die Mengen an Interferon, die bisher für wissenschaftliche und therapeutische Untersuchungen eingesetzt werden konnten, wurden aus Fibroblasten- und Lymphocyten-Kulturen gewonnen. Es gelang aber mit beiden Verfahren nicht, größere oder auch nur für einzelne Fragestellungen ausreichende Mengen an Interferon herzustellen. Dies ist zugleich der Grund dafür, daß der Erkenntnisstand auf dem Interferon-Gebiet nur sehr langsam voranschreitet und daß nur sehr wenige der dringend gewünschten Untersuchungen auf dem Krebsgebiet durchgeführt werden konnten. Es ist somit unabdingbar, neue Wege in der Produktion von Interferon zu beschreiten.

In Research Disclosure july 1979, 361-362, wurde bereits ein Weg zur gentechnischen Herstellung von Interferon skizziert, wobei als Quelle auch menschliche Leukozytenzellen genannt sind. Man gewinnt hierbei mRNA in reiner oder teilweise reiner Form, überführt sie in cDNA, komplettiert sie in dsDNA, baut diese in ein Plasmid ein, transformiert dieses in E. coli und identifiziert die Klone, die Interferon exprimieren, mit Hilfe von Antikörpern.

Ein ähnliches Verfahren ist in Nature 238 (1980) 323 beschrieben. Aus Virus-stimulierten menschlichen Lymphozyten wurde eine rohe Mischung von mRNA extrahiert, wobei jedoch nicht angegeben ist, wie diese Extraktion durchgeführt wurde. Die mRNA wurde in cDNA übergeführt, die in pBR 322 eingebaut und in E. coli transformiert wurde. Durch Extraktion der Plasmid-DNA wurden diejenigen Bakterien in einem biologischen Test ermittelt, die das Interferon-Gen enthielten. Die Bakterien produzierten ein Interferon, das weitgehend dieselben biologischen und chemischen Eigenschaften wie authentisches menschliches Lymphozyten-Interferon aufwies.

Die Erfindung betrifft ein Verfahren zur Herstellung eines Polypeptids, das die Aminosäuresequenz von menschlichem Leukozyten-Interferon ganz oder teilweise enthält, durch Gewinnung von mRNA aus zur Interferonproduktion angeregten Leukozyten, die in cDNA transkribiert, in dsDNA komplettiert, diese in ein Plasmid eingebaut, in E. coli transformiert wird und die Klone, die die gewünschten Proteine exprimieren, mit immunologischen Methoden festgestellt werden, das dadurch gekennzeichnet ist, daß aus den angeregten Fibroblasten RNA gewonnen und daraus die gesamte mRNA isoliert und eingesetzt wird. In einem bevorzugten Verfahren werden die Klone durch die Bindung von radioaktiven Antikörpern an diese Proteine mit nachfolgender Autoradiographie nachgewiesen.

Zur Erreichung des erfindungsgemäßen Zieles kann man in folgender Weise verfahren, wobei der aufgezeichnete Weg beispielhaft für mehrere begangene steht.

Menschliches Leukozyten-Interferon enthaltende Zellen werden denaturiert, und die RNA wird als Niederschlag nach einer Cäsiumchloridzentrifugation gewonnen. Nach weiterem Umfällen wird aus dieser RNA die mRNA über eine Oligo-dT-Cellulosesäule abgetrennt. Die gewonnene mRNA wird mit Hilfe des Enzyms Reverse Transcriptase zu einem RNA-DNA-Doppelstrang komplettiert. Nach Verdauung des RNA-Stranges wird der DNA-Strang (die komplementäre DNA = cDNA) mit Reverser Transkriptase oder dem Enzym Polymerase 1 zum DNA-DNA-Doppelstrang (dsDNA) komplettiert.

Diese doppelsträngige DNA muß nun in ein Plasmid eingebaut werden. Dazu ist die Verlängerung des 3'-Endes der DNA z. B. mit dCMP-Resten notwendig. Das Plasmid (z.B. pBR 322) wird mit der Restriktionsnuklease Pstl aufgeschnitten und z. B. mit dGMP-Resten verlängert. Bei Zusammengabe der so verlängerten DNA mit dem entsprechend verlängerten Plasmid erfolgt Basenpaarung zwischen DNA und Plasmid. Dieses zirkularisierte Molekül wird dann in Mikroorganismen (vor allem Bakterien, vorzugsweise E. coli, wie E. coli K 12 oder $\chi$ 1776) transformiert; die noch nicht geschlossenen Bindungen werden durch die Enzyme des Mikroorganismus kovalent geknüpft. Die transformierten Mikroorganismen, werden auf Agarplatten ausplattiert und auf Antibiotikaresistenzen, die durch das gewählte Plasmid und die eingesetzte Restriktionsnuklease gegeben sind, selektioniert.

Mit Hilfe immunologischer und biologischer Teste werden die Klone, die interferonhaltige Proteine exprimieren, herausgesucht. Diese Klone werden gezüchtet, die Mikroorganismenmasse abzentrifugiert, extrahiert und auf Interferongehalt getestet. Die durch Aufschluß der Interferon-produzierenden Klone gewonnene Lösung enthält Interferon-Protein.

Das erfindungsgemäß Verfahren kann in folgender Weise durchgeführt werden:

1. Verfahren zur Isolierung von RNA

a) Die Gewinnung von interferonproduzierenden lymphoblastoiden Zellen

Zellen der lymphoblastoiden Zellinie Namalvar werden in an sich bekannter Weise bis zu einer Zellkonzentration von 1 - 6 $\times$ 10$^6$ Zellen/ml vermehrt. Für die Interferoninduktion wurden die Zellen in frischem Medium aufgenommen und bei einer Konzentration von 1 - 10 $\times$ 10$^6$ Zellen/ml mit Newcastle Disease Virus oder Sendaivirus infiziert (Multiplizität der Infektion: > 1,0). Nach 4 - 24 stündiger Inkubation bei 37°C werden die Zellen abzentrifugiert.

b) Gewinnung von interferonproduzierenden Leukozyten

Zur Gewinnung von interferonproduzierenden Leukozyten wurden Leukozytensuspensionen aus dem Blut gesunder Spender gewonnen. Hierzu wurde die weiße Zellschicht an der Grenze zwischen Erythrozyten und Serum von zentrifugierten Blutproben in geeigneten Pufferlösungen suspendiert und durch fraktioniertes Sedimentieren, z. B. Absetzen bei 1 g oder niedertourige Zentrifugation, von Erythrozyten und Thrombozyten getrennt. Anschließend wurden die Leukozyten in einem üblichen Nährmedium suspendiert und wie oben beschrieben induziert.

c) Gewinnung von RNA

Die erhaltenen Zellen werden in "denaturierendem Medium" (4 M Guanidiniumthiocyanat, 1 M Mercaptoäthanol, 0,15 M Natriumacetat - pH 5,5) aufgenommen und 1 Minute bei 0°C mit dem Ultra-Turrax homogenisiert. Das Homogenat wird 15 Minuten bei 4°C mit 20 000 Upm zentrifugiert.

Der Überstand wird dann auf eine Lösung ("Kissen") aus 5 ml 5,7 M CsCl, 10 mM Tris-hydroxiaminomethan (Tris), 10 mM Äthylendiamintetraessigsäure (EDTA) vom pH 7,6 geschichtet und in einem Beckman-SW 27 Rotor 36 Stunden bei 20°C und 22 000 UpM zentrifugiert.

Es wurde gefunden, daß, im Gegensatz zu Angaben in der Literatur, ein Zusatz von CsCl zu dem Lysat vermieden werden muß, um eine möglichst vollständige Abtrennung der mRNA im folgenden Reaktionsschritt zu erreichen. Nach beendeter Zentrifugation werden die Polyallomer-Röhrchen in flüssigem Stickstoff eingefroren und der Boden des Röhrchens, auf dem sich der RNA-Niederchlag befindet, abgeschnitten. Der Niederschlag wird in einer Lösung aus 10 mM Tris, 10 mM EDTA und 1 % "Sarkosyl" (N-Lauryl-Sarkosin-Na-Salz) vom pH 7,6 aufgenommen und die Suspension bei 20 000 UpM 20 Minuten abzentrifugiert. Der erhaltene Niederschlag wird nochmals in dem gleichen Puffer aufgenommen, 5 Minuten auf 65°C erwärmt und erneut zentrifugiert. Die vereinigten Überstände werden 0,3 M an Natriumacetat gemacht, mit dem 2,5-fachen Volumen an Äthanol versetzt und bei -20°C aufbewahrt.

2. Gewinnung von mRNA

Die RNA wird durch Abzentrifugation aus der äthanolischen Lösung (20 000 UpM, 30 Minuten, -5°C) abgetrennt und in 0,5 M NaCl, 10 mM Tris pH 7,5 gelöst. Diese Lösung wird auf eine Oligo-dT-Cellulose-Säule (Hersteller: Collaborative Research, Type 3), die mit dem gleichen Puffer äquilibriert ist, aufgetragen und mit 1 mM Tris pH 7,6 oder destilliertem Wasser eluiert. Die Elution der Poly-A-haltigen RNA (mRNA) kann durch Extinktionsmessung bei 260 nm verfolgt werden. Die so erhaltene mRNA wird mit Natriumacetat bis zu einer Endkonzentration von 0,3 M versetzt, das 2,5-fache Volumen an Äthanol zugegeben und die Lösung bei -20°C aufbewahrt.

3. Gewinnung der cDNA

a) Gewinnung von Einzelstrang-cDNA

Die Übertragung der mRNA in die entsprechende DNA (cDNA) erfolgt mit Hilfe des Enzyms Reverser Transcriptase. Der Inkubationsansatz enthält: 50 mM Tris, pH 8,3, 10 mM $MgCl_2$, 30 mM 2-Mercapto-äthanol, 0,5 mM an allen 4 üblicherweise vorkommenden Desoxyribonukleosidtriphosphaten (entsprechende Triphosphate von Adenin, Guanin, Cytosin und Thymidin), 100 μg/ml Oligo-$dT_{12-18}$ (Hersteller: Boehringer Mannheim) etwa 100 μg/ml polyadenylierte RNA und 800 Einheiten/ml Reverser Transcriptase (Hersteller: Life Science Inc., St. Petersburg, USA). Zur Verfolgung der Reaktion kann ein in α-Stellung mit [32]P-markiertes Desoxyribonukleosidtriphosphat (spez. Aktiv.: 50Ci/mMol) zum Ansatz gegeben werden. Das Gemisch wird 60 Minuten bei 42°C inkubiert, worauf durch Zusatz von 20 mM EDTA die Reaktion abgestoppt wird. Die Lösung wird mit einem gleichen Volumen mit Wasser gesättigtem Phenol extrahiert, das Phenol durch Ausschütteln mit Äther entfernt und Reste Äther mit Stickstoff abgedampft. Nicht eingebaute Desoxyribonukleosidtriphosphate werden durch Säulenchromatographie an Sephadex G50 in 10 mM Tris, pH 9,0, 100 mM NaCl, 1 mM EDTA abgetrennt. Die eluiierte cDNA wird durch Zusatz von 0,3 M Natriumacetat und dem 2,5-fachen Volumen Äthanol ausgefällt.

Nach Zentrifugation wird der Niederschlag in 0,1 M NaOH aufgenommen und 20 Minuten bei 70°C inkubiert oder in 0,3 M NaOH über Nacht bei Raumtemperatur inkubiert. Das Gemisch wird mit 1 M HCl und 1 M Tris auf pH 7,6 gebracht.

b) Bildung von Doppelstrang-cDNA (dsDNA)

Zur Synthese des zweiten DNA-Stranges kann wiederum das Enzym Reverse Transkriptase oder das Enzym Polymerase I verwendet werden.

Bildung der dsDNA mit Reverser Transcriptase:

Der Inkubationsansatz (pH 8,3) enthält 50 mM Tris, 10 mM $MgCl_2$, 30 mM 2-Mercaptoäthanol, 0,5 mM der oben genannten 4 Desoxyribonukleosidtriphosphate, 50 μg/ml cDNA und 800 Einheiten/ml Reverser Transcriptase. Die Reaktion wird 120 Minuten bei 42°C durchgeführt und durch Zusatz von EDTA bis zur Endkonzentration von 20 mM abgestoppt. Anschließend erfolgt die Phenol-Extraktion und Gelpermeationschromatographie an Sephadex G50 wie vorher beschrieben.

Bildung der dsDNA mit Polymerase I:

Der Inkubationsansatz (pH 6,9) enthält 200 mM Hepes, 0,5 mM der obengenannten 4 Desoxyribonukleosidtriphosphate, 10 mM $MgCl_2$, 30 mM 2-Mercaptoäthanol und 70 mM KCl. Durch Zusatz von [32]P-markierten Desoxyribonukleotiden (an den α-Positionen) zum Inkubationsansatz kann die Reaktion verfolgt werden. Die Reaktion wird mit 800 Einheiten/ml Polymerase I gestartet und 2 Stunden bei 15°C durchgeführt. Durch Zusatz von 0,1 % Natriumdodecylsulfat und 100

μg/ml RNA wird die Reaktion abgestoppt. Die Lösung wird wie beschrieben extrahiert. Die nach der Extraktion erhaltene Lösung wird auf eine Sephadex G50 Säule aufgetragen und die Chromatographie mit 10 mM Tris pH 9,0, 100 mM NaCl und 1 mM EDTA durchgeführt. Die eluierte DNA kann durch die Radioaktivität bestimmt werden und wird durch Zusatz von 0,3 M Natriumacetat und 2,5-fachem Volumen Äthanol gefällt. Der Äthanol-Niederschlag wird in einer Lösung (pH 4,5) aus 300 mM NaCl, 30 mM Natriumacetat und 3 mM $ZnCl_2$ aufgenommen und mit 1500 Einheiten/ml S1 Nuklease (Boehringer Mannheim) versetzt. Die Reaktion wird nach einer Stunde bei 37°C durch Zusatz von EDTA bis zu einer Endkonzentration von 20 mM beendet. Anschließend erfolgt Extraktion mit Phenol und Fällung mit Äthanol wie beschrieben.

c) Gewinnung von cDNA in einem "Eintopfverfahren"

Alternativ zu den beschriebenen Reaktionen kann die dsDNA aus der mRNA auch in einem "Eintopfverfahren" gewonnen werden. Dabei wird die Reaktion der Reversen Transcriptase wie beschrieben durchgeführt, zum Inkubationsansatz wird allerdings noch 140 mM KCl hinzugefügt. Nach beendeter Reaktion wird die Probe 4 Minuten auf 100°C erhitzt und der gebildete Niederschlag abzentrifugiert. Zu dem Überstand wird das gleiche Volumen an 0,4 M Hepes Puffer (pH 6,9) gegeben, wobei die oben genannten 4 Desoxyribonukleotide in dem Puffer 0,5 mM vorhanden sind. Durch Zugabe von 800 Einheiten/ml Polymerase I wird die Reaktion wie beschrieben bei 15°C durchgeführt und durch Zusatz von Natriumdodecylsulfat und RNA abgestoppt. Anschließend wird wie unter 3.b) "Bildung der dsDNA mit Polymerase I" weiter verfahren.

4. Verlängerung der 3'-Enden von cDNA mit dCTP (Desoxicytosintriphosphat)

Zum Einbau in das Plasmid ist die Verlängerung des 3'-Endes der DNA mit einem der oben genannten 4 Desoxynukleotide notwendig. Ferner werden die 3'-Enden des geschnittenen Plasmids mit dem komplementären Desoxynukleotid verlängert. Bei Zusammengabe der DNA mit dem Plasmid erfolgt dann eine Basenpaarung zwischen DNA und Plasmid. Dieses wieder zirkularisierte Molekül kann zur Transformation von Bakterien eingesetzt werden; die noch nicht geschlossenen Bindungen werden durch ein Enzymsystem im Bakterium kovalent verknüpft.

Beispielsweise können die 3'-Enden der cDNA mit dCMP-Resten (Desoxicytosinmonophosphatreste) und das Plasmid mit dGMP-Resten (Desoxiguanidinmonophosphat-Reste) verlängert werden. Bei Verwendung dieser Desoxynukleotide in der beschriebenen Weise und bei Öffnung des Plasmids durch das Restriktionsenzym Pst I wird nach Einbau der fremden DNA eine Pst I-Spaltstelle an jeder Insertionsstelle neu gebildet, so daß nach Vermehrung des Plasmids die insertierte DNA zur weiteren Untersuchung leicht wieder mit dem Restriktionsenzym Pst I herausgeschnitten werden kann.

Der Niederschlag aus der Alkohol-Fällung nach Abbau mit S1 Nuklease wird in einer wäßrigen Lösung (pH 6,7) aus 30 mM Tris, 1 mM $CoCl_2$, 140 mM Kalium-Kacodylat, 150μM dCTP, 100μg autoklavierten Gelatinehydrolisats und 0,1 M Dithioerythrit gelöst. Zur Verfolgung der Reaktion sollte $^{32}$P-markiertes dCTP verwendet werden. Die Reaktion wird durch Zusatz von Terminaler Desoxynukleotidyl-Transferase gestartet und für 10 Minuten bei 37°C durchgeführt. Nach Ablauf der Reaktionszeit wird die Probe in Eis gestellt und durch Radioaktivitätsmessung im mit Trichloressigsäure fällbaren Niederschlag die Anzahl der eingebauten dCMP-Reste bestimmt. Bei der Reaktion sollten etwa 10 % der ursprünglich vorhandenen Nukleotide addiert worden sein; wenn dies nicht der Fall ist, kann durch Zusatz von weiterem Enzym die Reaktion fortgesetzt werden. Falls eine zu große Anzahl von dCMP-Resten addiert wurde, kann die entstandene Kette mit Hilfe des Enzyms S1 Nuklease verkürzt werden.

5. Verfahren zur Integration von DNA in ein Plasmid

Ein geeignetes zirkuläres Plasmid, beispielsweise pBR 322, wird mit einer Restriktionsendonuklease geschnitten, welche nur eine Sequenz auf dem Plasmid erkennt. Günstig ist es, wenn diese Spaltstelle hinter einem starken oder induzierbaren Promotor liegt und/oder so lokalisiert ist, daß eine Antibiotikaresistenz beeinflußt wird.

Das solchermaßen linearisierte Plasmid wird dann an den 3'-Enden um eines der vier Desoxynukleotide verlängert.

Dies wird z. B. folgendermaßen durchgeführt: 30 μg Plasmid werden mit 50 Einheiten Pst I Restriktionsendonuklease in Gegenwart von 50 mM NaCl, 6 mM Tris, 6 mM $MgCl_2$, 6 mM 2-Mercaptoäthanol und 0,1 mg/ml Gelatine für 40 Minuten bei 37°C und einem pH-Wert von 7,5 inkubiert. Anschließend wird mit Phenol und Äther wie beschrieben extrahiert und das geschnittene Plasmid mit Äthanol in Gegenwart von 0,3 M Natriumacetat gefällt. Die Verlängerung der 3'-Enden des Plasmids mit dGTP (Desoxiguanidintriphosphat) erfolgt analog zu der oben beschriebenen Verlängerung der cDNA mit dCTP.

Die verlängerte Plasmid-DNA wird dann in einer Lösung (pH 8,0) aus 0,1 M NaCl, 10 mM Tris und 1 mM EDTA mit der verlängerten ds cDNA vermischt, für 2 Minuten auf 56°C erwärmt, 2 Stunden bei 42°C inkubiert und dann langsam auf Raumtemperatur abgekühlt. Die solchermaßen erhaltene hybride DNA wird dann zur Transformation von E. coli verwendet.

6. Klonierung des Hydridplasmids in E. coli

Die Bakterien, beispielsweise E. coli χ 1776, wurden bei 37°C in 50 ml eines üblichen Nährmediums bis zu einer optischen Dichte von $A_{600} = 0,5 - 0,6$ wachsen gelassen, sedimentiert, einmal mit 10 mM Tris pH 7,5 gewaschen und dann in 40 ml einer Lösung aus 75 mM CaCl$_2$, 5 mM MgCl$_2$ und 5 mM Tris vom pH 8,0 resuspendiert und 20 Minuten in Eis inkubiert. Dann wurden die Zellen sedimentiert und in 2 ml 75 mM CaCl$_2$, 5 mM MgCl$_2$, 5 mM Tris pH 8,0 resuspendiert.

0,2 ml dieser Bakteriensuspension wurden dann mit 0,1 ml Hybrid-DNA-Lösung vermischt und 45 Minuten auf Eis inkubiert. Dann wurde für 90 Sekunden auf 42°C erwärmt und anschließend mit 0,2 ml Nährmedium vermischt.

50 µl-75 µl dieser Suspension wurden dann auf Tetracyclin und Ampicillin enthaltende Agarplatten ausplattiert und auf Antibiotikaresistenz selektioniert.

### 7. Isolierung von Stämmen, die interferonenthaltende Proteine produzieren

#### a) Immunologischer Nachweis

Mit Hilfe eines von Broome und Gilbert (Broome, S. und Gilbert, E. PNAS 75, 2746, 1978) entwickelten Testsystems wurden die Klone auf die Expression interferonenthaltender Proteine untersucht. Klone, die interferonenthaltende Proteine exprimieren, werden durch die Bindung von radioaktivem Antikörper an diese Proteine mit nachfolgender Autoradiographie dadurch erkenntlich, daß der Röntgenfilm an dieser Stelle geschwärzt wird. Dazu wurden bis zu 50 Klone pro Nitrocellulosefilter für 2 Tage bei 37°C wachsen gelassen. Dann wurden die Filter auf einen Agarblock gelegt, welcher 1 mg/ml Lysozym enthielt; auf die Bakterienkolonie wurde eine mit Interferonantikörper beschichtete PVC-Folie aufgebracht, und dann wurde für 2 - 3 Stunden bei 4°C inkubiert. Die PVC-Folie wurde anschließend für 15 Stunden bei 4°C in einer Lösung von [132]Jod-markiertem Interferonantikörper inkubiert. Die spezifische Aktivität der Lösung war ca. 1 × 10$^6$ cpm/ml. Nach dem Waschen und Trocknen der Folien wurden diese autoradiographiert.

#### b) Biologischer Nachweis

Die Klone wurden in 50 ml eines üblichen Nährmediums bei 37°C über Nacht gezüchtet. Dann wurden die Bakterien sedimentiert, zweimal mit kalten 10 mM Tris pH 8,0, 30 mM NaCl gewaschen und in 20 % Saccharose in 30 mM Tris pH 8,0 1 mM EDTA resuspendiert. Es wurde dann bei Raumtemperatur für 10 Minuten geschüttelt, sedimentiert, in eiskaltem H$_2$O resuspendiert und im Eisbad für 10 Minuten inkubiert. Die Bakterien wurden dann sedimentiert und der Überstand in an sich bekannter Weise in einem Virusinbihitionstest auf den Gehalt an interferonhaltigem Protein getestet.

### 8. Gewinnung von Interferon-Protein

Die Klone, die in den verschiedenen Tests Interferonaktivität zeigen, werden in den üblichen Nährmedien gezüchtet, die Bakterien werden abzentrifugiert und mit wäßrigen Puffern extrahiert. Die Extraktionslösung enthält das Interferonprotein.

## Patentansprüche

1. Verfahren zur Herstellung eines Polypeptids, das die Aminosäuresequenz von menschlichem Leucozyteninterferon ganz oder teilweise enthält, durch Gewinnung von mRNA aus zur Interferonproduktion angeregten Leucozyten, die in cDNA transcribiert, in dsDNA komplettiert, diese in ein Plasmid eingebaut, in E. coli transformiert wird und die Klone, die die gewünschten Proteine exprimieren, mit immunologischen Methoden festgestellt werden, <u>dadurch gekennzeichnet</u>, daß aus den angeregten Leucozyten RNA gewonnen und daraus die gesamte mRNA isoliert und eingesetzt wird.

2. Verfahren nach Anspruch 1, <u>dadurch gekennzeichnet</u>, daß die Klone durch die Bindung von radioaktiven Antikörpern an diese Proteine mit nachfolgender Autoradiographie nachgewiesen werden.

## Claims

1. A process for the preparation of a polypeptide which contains the aminoacid sequence of human leucocyte interferon totally or partially, by obtaining mRNA form leucozytes which have been induced to produce interferon, transcribing the mRNA into cDNA, completing it into dsDNA, inserting the latter into a plasmid, transforming the plasmid into E. coli and detecting those clones which express the desired proteins, <u>characterized by</u> obtaining RNA form the induced leucozytes, isolating therefrom the total mRNA and using it in this process.

2. A process according to claim 1, <u>characterized in that</u> the clones are detected by binding of radioactive antibodies onto these proteins, followed by autoradiography.

## Revendications

1. Procédé pour la préparation d'un polypeptide qui contient en totalité ou en partie la séquence d'acides aminés de l'interféron de leucocyte humain, par obtention d'ARNm à partir de leucocytes stimulés pour la production d'interféron, lequel ARNm est transcrit en ADNc, complete en ADNdd (double brin), on incorpore celui-ci dans un plasmide, on le transforme dans E. coli et on identifie par des méthodes immunologiques les clones qui expriment les protéines recherchées, caractérisé en ce que l'on extrait de l'ARN des leucocytes stimulés et que l'on isole à partir de celui-ci l'ensemble de l'ARNm et qu'on l'utilise ainsi.

2. Procédé selon la revendication 1, caractérisé en ce que l'on identifie les clones par la fixation d'anticorps radioactifs sur ces protéines avec autoradiographie subséquente.